# EUROPEAN PATENT APPLICATION

(11) **EP 4 089 077 A1**
(43) Date of publication of application: **16.11.2022**
(21) Application number: 21173009.8
(22) Date of filing: 10.05.2021
(51) Int. Cl.: C07D 215/50, C07D 401/04, C07D 405/04, C07D 409/04, A61K 31/4709, A61P 35/00

(54) **NOVEL QUINOLINE DERIVATIVES AND USES THEREOF**

(71) Applicant: Istituto Europeo di Oncologia S.r.l., 20121 Milano (MI) (IT); Università degli Studi di Roma "La Sapienza", 00185 Roma (IT)
(72) Inventor: SILVESTRI, Romano, 00152 ROMA (IT); LA REGINA, Giuseppe, 00041 Albano Laziale (RM) (IT); COLUCCIA, Antonio, 00032 Carpineto Romano (RM) (IT); MINUCCI, Saverio, 20090 Opera (MI) (IT); DE WULF, Peter Arthur Irene, 38123 Povo (TN) (IT)
(74) Representative: Bartorelli, Luisa

(57) **Abstract**

The invention refers to new quinoline-4-carboxamide derivatives, pharmaceutical compositions containing thereof and the use of the same as inhibitors of Ndc80 kinetochore subcomplex-MT binding and of kinetochore-MT binding, more preferably for use in the treatment of cell proliferative disorders including cancers and drug-resistant cancer.

## Description

### Field of the invention

The aim of this invention is to provide novel inhibitors of Ndc80 kinetochore subcomplex-microtubule binding.

Another aim of this invention is to provide compounds and pharmaceutical compositions thereof to treat cell proliferative disorders including cancers and multidrug-resistant cancers.

Another aim of this invention is to provide compounds and compositions thereof to study kinetochore biology and the cell cycle division process.

### Background of the invention

Following chromosome replication during cell division, each chromosome and its copy are bound together by cohesion ring complexes, forming sister chromatid pairs. Each chromosome within each sister chromatid pairs next recruits >100 proteins (singularly or as small preformed subcomplexes) onto their centromere sequence to form a large protein structure known as the kinetochore (Musacchio A. & Desai A. (2017). Biology (Basel), 6:5). Both kinetochores then bind (bi-orient) their underlying sister chromatid pair to the microtubules (MTs) of the mitotic spindle, which are α,β-tubulin polymers that extend from two opposite MT-organising centers named centrosomes. Following cohesion cleavage by the enzyme separase, MT depolymerisation moves each chromosome -firmly attached by the kinetochore to the MT tip- towards the adjacent centrosome. This activity produces two daughter cells that carry a correct number of chromosomes (euploidy). Cells suffering from erroneous kinetochore activity generate daughter cells with e a deviant number of chromosomes, known as aneuploidy. Aneuploidy is an established hallmark of cancer, indicating that chromosome missegregation and the consequent chromosome number imbalance promotes cancerogenesis and/or cancer progression (Knouse K.A. et al. (2017) Annu Rev Cancer Biol, 1: 335-54). To prevent aneuploidy, cells have evolved the spindle assembly checkpoint (SAC), a protein signaling complex that binds to the kinetochore-spindle interface to monitor sister chromatid bi-orientation to the spindle MTs. When the SAC senses that even one sister chromatid pair is un/misbound to the spindle it will halt cell division, allowing the cell to correctly align each sister chromatid pair. Only when the SAC is satisfied will it allow cell division and chromosome transmission to proceed. However, if the cell fails to bi-orient each of its sister chromatids (e.g., because of defective kinetochore activity or kinetochore-MT binding), then the SAC will keep the cells arrested, a condition that will culminate in the death of the cell by apoptosis (Corbett K.D. (2017). Prog Mol Subcell Biol, 56: 429-55).

The Ndc80 kinetochore subcomplex lies in the outer region of the kinetochore to both bind the kinetochore to MTs and to recruit the SAC. This subcomplex exists as an elongated, rod-like structure that comprises four subunits existing as an upper Ndc80 (Hec1)-Nuf2 dimer, and a lower Spc24-Spc25 dimer. The Ndc80 kinetochore subcomplex is recruited to the kinetochore by its Spc24-Spc25 dimer, which also binds the SAC. The Ndc80 (Hec1)-Nuf2 dimer mediates kinetochore-spindle MT binding (Ustinov N.B. et al. (2020). Biochemistry (Moscow), 85:448-65). Overexpression solely of the Ndc80 subunit (Ndc80 was originally named Heel = Highly Expressed in Cancer 1), interferes with MT-binding and SAC signaling activities, resulting in unbound chromosomes and aneuploidy, prorogating in hyper-proliferation and cancer formation, as shown in mouse cancer models (Diaz-Rodriguez E. et al. (2008). Proc Natl Acad Sci USA, 105: 16719-24). With very high frequency, cancers covering the entire cancer spectrum overexpress Ndc80 (e.g., Chen X. et al. (2019). Int J Clin Exp Pathol, 12:1233-47; Yan X. et al. (2018). Cancer Medicine, 7:420-32); the other subunits of the Ndc80 kinetochore subcomplex (e.g., Sun Z.-Y. et al. (2020). J Cancer, 11:2921-34; Xu W. et al. (2019). Int J Mol Med, 44:390-404; Zhou J. et al. (2017). Oncotarget, 8:65469-80; Wang Q. et al. (2019). Aging, 11:5689-5703) as well as myriad other kinetochore proteins -often in a coordinated manner- (Thiru P. et al. (2014). Mol Biol Cell, 25:1983-94), resulting in structurally and functionally unfit kinetochores that underlie aneuploidy and cancerogenesis. Overexpressed kinetochore subunits (including the four components of the Ndc80 kinetochore subcomplex) have become prognostic biomarkers that correlate with low cancer patient survival and a poor response to radio- and chemotherapies (Zhang W. et al., (2016) Nature Commun, 7:12619). Genetic screens have revealed strong lethal interactions between kinetochore proteins/regulators, and many cancer-driving mutations (e.g., KRAS, p53, PTEN) (Luo J. et al. (2009). Cell, 137:835-48; Wang X. & Simon R. (2013). BMC Medical Genomics, 6:30; Brough R. et al. (2011). Cancer Discov, 1:260-73). Hence, targeting kinetochore activity in hitherto untreatable or therapy-resistant cancer cells represents a new approach to clinically eradicate these cancers. This strategy can be realised using small compounds that prevent the Ndc80 kinetochore subcomplex from binding the underlying kinetochore and chromosome to the spindle MTs. As these ligands do not affect kinetochore assembly or its structural integrity, they will ensure SAC proficiency. As a consequence, the drug-treated cells will arrest and undergo apoptosis therewith selectively eradicating the cancer cells characterised by specific cancer-driving mutation(s). In addition, cancer cells overexpressing or mutated in kinetochore subunits will also be selectively arrested by the SAC in the presence of Ndc80 kinetocore subcomplex -MT inhibitors, hence preventing the structurally/functionally inadequate kinetochores to misalign and mistransmit their replicated genome (aneuploidy). Since aneuploidy is extremely rare in normal tissues, a treatment that selectively eliminates aneuploid cells would be highly successful against a broad spectrum of cancers while generating few side effects on normal cells. Drug-targeting Ndc80 kinetochore subcomplex-MT binding, for example as part of a personalized therapy for patients suffering from over/reduced expression of or mutations in one or more kinetochore subunits, and/or kinetochore regulating factors and/or kinetochore modifying enzymes, and/or proteins contributing to aneuploidy, represents a new therapeutic approach to selectively eradicate genetically unstable, proliferating cancer cells, including those that do not respond to current therapies, hence expanding treatment options for oncologists. In addition, such compounds will allow for as-yet inconceivable studies of kinetochore and chromosome biology that promise to advance our understanding of the cell division process and its regulation.

### Description of the invention

The present invention concerns novel quinoline derivatives and the use thereof in the medical field. In particular, the invention concerns novel quinoline-4-carboxamide derivatives and bioisosteric derivatives thereof and their use as a medicament.

The inventors have discovered that quinoline-4-carboxamide derivatives inhibit the binding of the Ndc80 kinetochore subcomplex to MTs (and hence the underlying kinetochore structure); therefore, the present invention preferably concerns novel quinoline derivatives as below discussed for use as inhibitors of Ndc80 kinetochore subcomplex-MT binding and of kinetochore-MT binding, more preferably for use in the treatment of cell proliferative disorders including cancers and drug-resistant cancers. The inhibitors can also be used in a research setting to study kinetochore and cell cycle biology in a broadest variety of model organisms.

It is therefore object of the present invention of novel quinoline-4-carboxamide derivatives and bioisosteric derivatives thereof of formula (I): 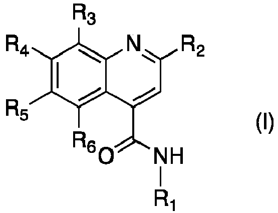 wherein
R₁ is selected among hydrogen, phenyl, heterocycle, benzofused heterocycle, optionally substituted at one or more position(s) with following same or different groups: alkyl, C₃-C₁₀ cycloalkyl, C₂-C₆ alkenyl, C₂-C₆ alkynyl, C₁-C₆ alkoxy, C₂-C₆ alkoxycarbonyl, 3-10 membered heterocycloalkyl, halogen, -CN, NH₂, OH, NO₂, NHR₁' NR₁'R₁" OR₁' where R₁', R₁" is alkyl, C₃-C₁₀ cycloalkyl, C₂-C₆ alkenyl, C₂-C₆ alkynyl, C₁-C₆ alkoxy, C₂-C₆ alkoxycarbonyl, 3-10 membered heterocycloalkyl or aromatic ring selected from phenyl, phenoxy, benzyl, benzyloxy, naphthyl, naphthyloxy, biphenyl or heterocycle, said aromatic ring optionally substituted at one or more position(s) with following same or different groups: alkyl, C₂-C₆ alkenyl, C₂-C₆ alkynyl, C₁-C₆ alkoxy, C₂-C₆ alkoxycarbonyl, 3-10 membered heterocycloalkyl, halogen, -CN, -NH₂, -OH, -NO₂;
R₂ is selected among hydrogen, phenyl, heterocycle, benzofused heterocycle, optionally substituted at one or more position(s) with following same or different groups: alkyl, C₃-C₁₀ cycloalkyl, C₂-C₆ alkenyl, C₂-C₆ alkynyl, C₁-C₆ alkoxy, C₂-C₆ alkoxycarbonyl, 3-10 membered heterocycloalkyl, halogen, -CN, NH2, OH, NO₂, NHR₁' NR₁'R₁" OR₁' where R₁', R₁" is alkyl, C₃-C₁₀ cycloalkyl, C₂-C₆ alkenyl, C₂-C₆ alkynyl, C₁-C₆ alkoxy, C₂-C₆ alkoxycarbonyl 3-10 membered heterocycloalkyl or aromatic ring selected from phenyl, phenoxy, benzyl, benzyloxy, naphthyl, naphthyloxy, biphenyl or heterocycle, said aromatic ring optionally substituted at one or more position(s) with following same or different groups: alkyl, C₂-C₆ alkenyl, C₂-C₆ alkynyl, C₁-C₆ alkoxy, C₂-C₆ alkoxycarbonyl, 3-10 membered heterocycloalkyl, halogen, -CN, -NH₂, -OH, -NO₂;
R₃ is selected among hydrogen, alkyl, C₃-C₁₀ cycloalkyl, C₂-C₆ alkenyl, C₂-C₆ alkynyl, C₁-C₆ alkoxy, C₂-C₆ alkoxycarbonyl, 3-10 membered heterocycloalkyl, halogen, -CN, NH₂, OH, NO₂;
R₄ is selected among hydrogen, alkyl, C₃-C₁₀ cycloalkyl, C₂-C₆ alkenyl, C₂-C₆ alkynyl, C₁-C₆ alkoxy, C₂-C₆ alkoxycarbonyl, 3-10 membered heterocycloalkyl, halogen, -CN, NH₂, OH, NO₂;
R₅ is selected among hydrogen, alkyl, C₃-C₁₀ cycloalkyl, C₂-C₆ alkenyl, C₂-C6 alkynyl, C₁-C₆ alkoxy, C₂-C₆ alkoxycarbonyl, 3-10 membered heterocycloalkyl, halogen, -CN, NH₂, OH, NO₂;
R₆ is selected among hydrogen, alkyl, C₃-C₁₀ cycloalkyl, C₂-C₆ alkenyl, C₂-C₆ alkynyl, C₁-C₆ alkoxy, C₂-C₆ alkoxycarbonyl, 3-10 membered heterocycloalkyl, halogen, -CN, NH₂, OH, NO₂, preferably in their crystalline structure or polymorphic crystalline structure, pharmaceutically acceptable salt, racemate, diastereoisomer or enantiomer. Preferably, the pharmaceutically acceptable salt is a hydrate salt.

It is then object of the present invention novel quinoline-4-carboxamide derivatives and bioisosteric derivatives thereof of formula (I) as above disclosed, for use as a medicament.

It is further object of the present invention novel quinoline-4-carboxamide derivatives and bioisosteric derivatives thereof of formula (I) as above disclosed, for use in the treatment and/or prevention of a disease wherein the Ndc80 kinetochore subcomplex is involved, preferably for use as inhibitors of Ndc80 kinetochore subcomplex binding as well as kinetochore binding to the MTs of the mitotic spindle structure. According to one embodiment, the novel quinoline-4-carboxamide derivatives and bioisosteric derivatives thereof of formula (I) as above disclosed are for use in the treatment and/or prevention of cell proliferative disorders, preferably for use in the treatment of cancers and/or multi-drug resistant cancers. Cancers and/or multi-drug resistant cancers according to the invention are intended as including, but not restricted, to cancers characterized by the over- or underexpression of or mutations in one or more kinetochore subunits and/or kinetochore regulating factors and/or kinetochore modifying enzymes (Tate J.G. et al. (2019). Nucleic Acids Res, 47(D1):D941-D947c) and/or proteins contributing to aneuploidy, as well as to cancers caused by driver mutations in oncogenes or tumor suppressor genes, including -among many other- K/H/N-RAS, p53 or PTEN mutations.

Cancers and/or multi-drug resistant cancers according to the invention are then intended as including but not restricted to breast cancer, uterine cancer, brain cancer, lung cancer, liver cancer, kidney cancer, testicular cancer, pancreatic cancer, skin cancer, thyroid cancer, intestinal cancer, head and neck cancer, ovarian cancer, stomach cancer, colon cancer, bladder cancer, prostate cancer and urinary tract cancer, eye cancer, cancer of the central nervous system, oral and oropharyngeal cancers, HIV/AIDS-related cancers, hematopoietic and lymphoid cancers, and sarcomas of various tissues. More preferably, the novel quinoline-4-carboxamide derivatives and bioisosteric derivatives thereof of formula (I) as above disclosed are for use in the treatment of breast cancer, lung cancer, prostate cancer, bladder cancer, colon cancer.

The present invention also relates to a method to identify a subject to be treated with the novel quinoline-4-carboxiamide derivatives and bioisosteric derivatives of the present invention or combination thereof comprising in vivo or ex vivo detection in the genome of a sample from said patient a mutation in one or more genes encoding kinetochore subunits (e.g. including but not restricted to Ndc80, Nuf2, Spc24, Spc25, CENP-A->Z) and/or kinetochore regulating factors and/or kinetochore modifying enzymes (e.g. the SAC, kinetochore and centromere kinases and phosphatases, (de)SUMOylases, (de)acetylases, (de)methylasess, (de)ubiquitinases, (de)glycosylases, (de)lipidases, (de)glycosidases, etc.) or genetic alterations causing aneuploidy and cancer or that promote cancer development (Tate J.G. et al. (2019). Nucleic Acids Res, 47(D1): D941-D947)) and/or measuring expression level variation (over- or underexpression) compared to a proper control sample of one or multiple structural kinetochore subunits and/or kinetochore regulating factors and/or kinetochore modifying enzymes, and/or proteins contributing to aneuploidy and cancer.

Any known methods in the art (such as for example sequencing) may be used to detect said mutation in one or more genes encoding kinetochore subunits and/or kinetochore regulating factors and/or kinetochore modifying enzymes, or genetic alterations causing aneuploidy and cancer.

Measuring expression level variation of any of the above indicated kinetochore subunits and/or kinetochore regulating factors and/or kinetochore modifying enzymes and/or proteins contributing to aneuploidy and cancer, when misexpressed or mutated, may be performed by any means known in the art. Expression level variation is measured by comparing to a proper control that may be the level present in a healthy subject, the level measured before initiation of the treatment with inhibitors of the Ndc80 kinetochore subcomplex-MT binding or the levels measured after treatment with a gold standard. Preferably said patient suffers from cancer, and/or is resistant to standard-of-care cancer treatments.

In one other object of the present invention such method is used to monitor the outcome of the therapeutic treatment with the novel quinoline-4-carboxiamide derivatives and bioisosteric derivatives of the invention.

The term "heterocycle" is meant as a heterocyclic aromatic compound in which one or more of the carbon atom(s) in the backbone of the molecule has been replaced by an atom other than carbon. Typical hetero atoms include nitrogen, oxygen, and sulfur. In particular, heterocycles are pyrrole, thiophene, furan, pyridine, pirimidine

The term "benzofused heterocycle" is meant as a heterocyclic aromatic compound in which one or more side(s) are fused with a phenyl ring. In particular, benzofused heterocycles are indole, benzofuran and benzothiofene, quinoline, isoquinoline.

The term "C₂-C₆ alkenyl" is meant as a C2-C₆ chain containing at least one double bond between adjacent carbon atoms. Alkenyls include both cis and trans isomers. In particular, C₂-C₆ alkenyl are ethylenyl, propylenyl, 1-butenyl, 2-butenyl, isobutylenyl, 1-pentenyl, 2-pentenyl, 3-methyl- 1-butenyl, 2-methyl-2-butenyl, 2,3-dimethyl-2-butenyl.

The term "C₂-C₆ alkynyl" is meant as a C₂-C₆ chain containing at least one triple bond between adjacent carbon atoms. In particular, C₂-C₆ alkynyl are ethynyl, propynyl, pentynyl amd their isomers.

The term "C₁-C₆ alkoxy" is meant as a C₁-C₆ alkyl chain having and oxygen atom. In particular, C₁-C₆ alkoxy are methoxy, ethoxy, propoxy, butoxy, pentoxy, and their isomers.

The term "C₂-C₆ alkoxycarbonyl" is meant as a C₁-C₆ alkyl chain having OC=O group. In particular, C₂-C₆ alkoxycarbonyl are methoxycarbonylmethyl, ethoxycarbonylmethyl, ethoxycarbonylmethyl, ethoxycarbonylethyl.

The term "C₃-C₁₀ cycloalkyl" is meant as a saturated, cyclic hydrocarbon group having 3 to 10 carbon atoms. Any carbon atom may be substituted by one or more substituents among alkyl or halogen. In particular, C₃-C₁₀ cycloalkyl are cyclopropyl, cyclobutyl, cyclopentyl and cyclohexyl.

The term "3-10 membered heterocycloalkyl" is meant as a saturated, cyclic hydrocarbon group having 3 to 10 carbon atoms, wherein one or more carbon atom(s) are replaced by one or more heteroatom(s), such as nitrogen atom(s), oxygen atom(s) or sulfur atom(s), or a combination thereof, for example, but not limited to, one nitrogen atom and one oxygen atom, one nitrogen atom and one sulfur atom. In particular, 3-10 membered heterocycloalkyl are pyrrolidine, piperidine, piperazine, morpholine, thiomorpholine.

The term "halogen" refers to the atoms of fluorine, chlorine, bromine and iodine, included in group 17 of the periodic table of the elements.

The term "optionally substituted" may be meant that phenyl, heterocycle, benzofused heterocycle of the derivatives of this invention are optionally substituted at one or more position(s) with alkyl, C₃-C₁₀ cycloalkyl, C₂-C₆ alkenyl, C₂-C₆ alkynyl, C₁-C₆ alkoxy, C₂-C₆ alkoxycarbonyl, 3-10 membered heterocycloalkyl, halogen, -CN, NH₂, OH, NO₂, and substituted amino or hydroxy group.

The term "pharmaceutically acceptable salt or hydrate salt" refers to salts with inorganic or organic acids as well as with inorganic or organic bases which retain the original biological properties and do not show unwanted side effects. Salt with inorganic acid are prepared using acids such us, for example, hydrochloric acid, sulfuric acid, phosphoric acid, nitric acid; salt with organic acid are prepared using acids such us, for example, acetic acid, propionic acid, succinic acid, maleic acid, citric acid, ascorbic acid, pamoic acid, glycolic acid, stearic acid, phenylacetic acid, benzoic acid, salicylic acid, methansulfonic acid, benzenesulfonic acid, toluenesulfonic acid; salt with inorganic bases are prepared using bases such us, for example, sodium carbonate, caustic soda, ammonia or ammonium hydroxide; salt with organic bases are prepared using bases such us, for example, dimethylamine, piperidine, piperazione, morpholine, thiomorpholine.

The term "to treat" or "treatment" is meant as preventing, reducing, alleviating, curing or avoiding the symptoms or the condition, or inhibiting the progress of a disease that includes cell proliferative disorders (including cancers and multidrug-resistant cancers).

The term "quinoline-4-carboxamide" refers to compounds derived from the following chemical structure:

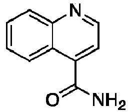

In an embodiment, the compounds of formula (I) are inhibitors of Ndc80 kinetochore subcomplex-MT binding: 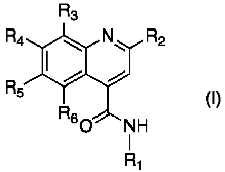 wherein
R₁ is selected among hydrogen, phenyl, heterocycle, benzofused heterocycle, optionally substituted at one or more position(s) with the following same or different groups: alkyl, C₃-C₁₀ cycloalkyl, C₂-C₆ alkenyl, C₂-C₆ alkynyl, C₁-C₆ alkoxy, C₂-C₆ alkoxycarbonyl, 3-10 membered heterocycloalkyl, halogen, -CN, NH₂, OH, NO₂, NHR₁' NR₁'R₁" OR₁' where R₁', R₁" is alkyl, C₃-C₁₀ cycloalkyl, C₂-C₆ alkenyl, C₂-C6 alkynyl, C₁-C₆ alkoxy, C₂-C₆ alkoxycarbonyl, 3-10 membered heterocycloalkyl or aromatic ring selected from phenyl, phenoxy, benzyl, benzyloxy, naphthyl, naphthyloxy, biphenyl or heterocycle, said aromatic ring optionally substituted at one or more position(s) with following same or different groups: alkyl, C₂-C₆ alkenyl, C₂-C₆ alkynyl, C₁-C₆ alkoxy, C₂-C₆ alkoxycarbonyl, 3-10 membered heterocycloalkyl, halogen, -CN, -NH₂, -OH, -NO₂;
R₂ is selected among hydrogen, phenyl, heterocycle, benzofused heterocycle, optionally substituted at one or more position(s) with the following same or different groups: alkyl, C₃-C₁₀ cycloalkyl, C₂-C₆ alkenyl, C₂-C₆ alkynyl, C₁-C₆ alkoxy, C₂-C₆ alkoxycarbonyl, 3-10 membered heterocycloalkyl, halogen, -CN, NH₂, OH, NO₂, NHR₁' NR₁'R₁" OR₁' where R₁', R₁" is alkyl, C₃-C₁₀ cycloalkyl, C₂-C₆ alkenyl, C₂-C₆ alkynyl, C₁-C₆ alkoxy, C₂-C₆ alkoxycarbonyl 3-10 membered heterocycloalkyl or aromatic ring selected from phenyl, phenoxy, benzyl, benzyloxy, naphthyl, naphthyloxy, biphenyl or heterocycle, said aromatic ring optionally substituted at one or more position(s) with following same or different groups: alkyl, C₂-C₆ alkenyl, C₂-C₆ alkynyl, C₁-C₆ alkoxy, C₂-C₆ alkoxycarbonyl, 3-10 membered heterocycloalkyl, halogen, -CN, -NH₂, -OH, -NO₂;
R₃ is selected among hydrogen, alkyl, C₃-C₁₀ cycloalkyl, C₂-C₆ alkenyl, C₂-C6 alkynyl, C₁-C₆ alkoxy, C₂-C₆ alkoxycarbonyl, 3-10 membered heterocycloalkyl, halogen, -CN, NH₂, OH, NO₂;
R₄ is selected among hydrogen, alkyl, C₃-C₁₀ cycloalkyl, C₂-C₆ alkenyl, C₂-C₆ alkynyl, C₁-C₆ alkoxy, C₂-C₆ alkoxycarbonyl, 3-10 membered heterocycloalkyl, halogen, -CN, NH₂, OH, NO₂;
R₅ is selected among hydrogen, alkyl, C₃-C₁₀ cycloalkyl, C₂-C₆ alkenyl, C₂-C₆ alkynyl, C₁-C₆ alkoxy, C₂-C₆ alkoxycarbonyl, 3-10 membered heterocycloalkyl, halogen, -CN, NH₂, OH, NO₂.
R₆ is selected among hydrogen, alkyl, C₃-C₁₀ cycloalkyl, C₂-C₆ alkenyl, C₂-C₆ alkynyl, C₁-C₆ alkoxy, C₂-C₆ alkoxycarbonyl, 3-10 membered heterocycloalkyl, halogen, -CN, NH₂, OH, NO₂;

In a particular embodiment, the compounds are characterised by the following formula (II) 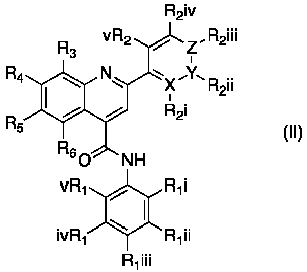 wherein
R₁i, R₁ii, R₁iii, R₁iv and R₁v are selected among H, alkyl, C₃-C₁₀ cycloalkyl, C₂-C₆ alkenyl, C₂-C₆ alkynyl, C₁-C₆ alkoxy, C₂-C₆ alkoxycarbonyl, 3-10 membered heterocycloalkyl, halogen, -CN, NH₂, OH, NO₂, mono or di substituted NH₂, substitured OH;
R₂i, R₂ii, R₂iii, R₂iv and R₂v are selected among H, alkyl, C₃-C₁₀ cycloalkyl, C₂-C₆ alkenyl, C₂-C₆ alkynyl, C₁-C₆ alkoxy, C₂-C₆ alkoxycarbonyl, 3-10 membered heterocycloalkyl, halogen, -CN, NH₂, OH, NO₂, mono or disubstituted NH₂, substitured OH;
R₃, R₄, R₅ and R₆ are selected among H, alkyl, C₃-C₁₀ cycloalkyl, C₂-C₆ alkenyl, C₂-C₆ alkynyl, C₁-C₆ alkoxy, C₂-C₆ alkoxycarbonyl, 3-10 membered heterocycloalkyl, halogen, -CN, NH₂, OH, NO₂, mono or disubstituted NH₂, substitured OH.
X, Y and Z are independently selected from CH, N. O or S;
X-Y and Y-Z is selected from N, NH, O, S, C=C, N=C, N=S.

In another embodiment, the compound of formula (I) has one of the following formulae:

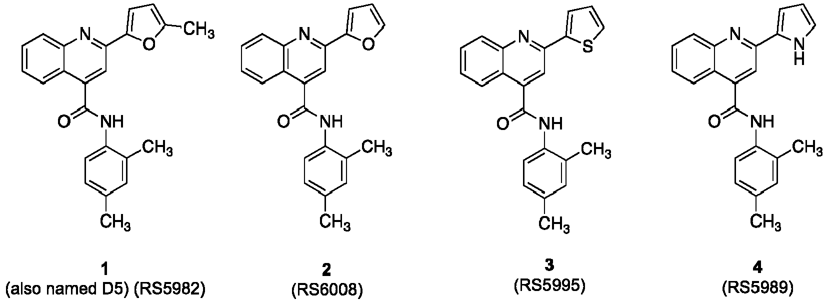

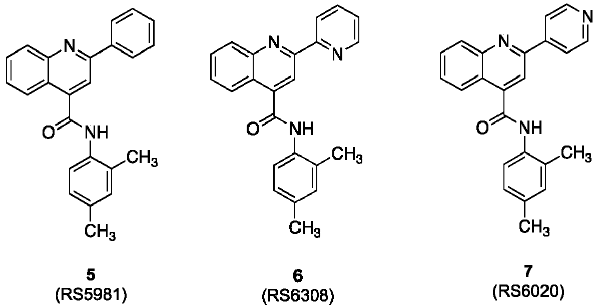

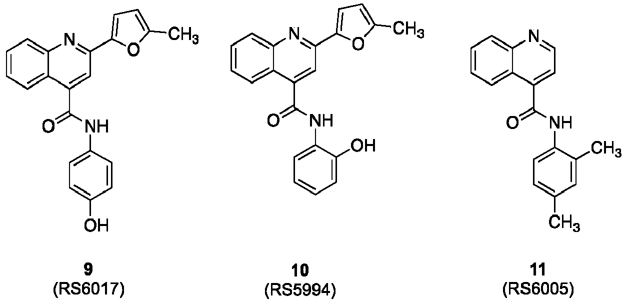

Preferably, the compound of formula (I) has the following formula:

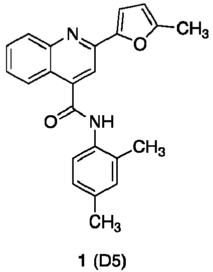

The present invention also relates to a pharmaceutical composition comprising at least one compound having formula (I) as above disclosed, in association with at least one pharmaceutically acceptable excipient.

The present invention further relates to a pharmaceutical composition comprising at least one compound having formula (I) as above disclosed, in association with at least one pharmaceutically acceptable excipient for use as a medicament.

The present invention further relates to a pharmaceutical composition comprising at least one compound having formula (I) as above disclosed in association with at least one pharmaceutically acceptable excipient for use in the treatment and/or prevention of a disease wherein the Ndc80 kinetochore subcomplex is involved, preferably for use as inhibitors of Ndc80 kinetochore subcomplex-MT binding. According to one embodiment, the pharmaceutical composition of the invention is for use in the treatment and/or prevention of cell proliferative disorders, preferably for use in the treatment of cancers and multi-drug resistant cancers, including but not restricted to breast cancer, uterine cancer, brain cancer, lung cancer, liver cancer, kidney cancer, testicular cancer, pancreatic cancer, skin cancer, thyroid cancer, intestinal cancer, head and neck cancer, ovarian cancer, stomach cancer, colon cancer, bladder cancer, prostate cancer and urinary tract cancer, eye cancer, cancer of the central nervous system, oral and oropharyngeal cancers, HIV/AIDS-related cancers, hematopoietic and lymphoid cancers, and sarcomas of various tissues.

The present invention also relates to a drug for use as defined above, including a compound having formula (I).

The pharmaceutical composition according to the invention may be both for veterinary and for human use.

The pharmaceutical composition according to the invention comprises or essentially consists of at least one compound having formula (I) as above defined, together with one or more pharmaceutically acceptable carriers. The pharmaceutical composition according to the invention eventually comprises at least one additional active ingredient. According to one embodiment, the pharmaceutical composition according to the invention comprises a compound having formula (I) as above disclosed as the sole active ingredient, without any additional active ingredient. In another embodiment, the at least one compound of formula (I) as above disclosed and the at least one additional active ingredient are used for combination therapy together in a single pharmaceutical composition or in different pharmaceutical compositions. In another embodiment, the pharmaceutical composition of the invention comprising at least one compound having formula (I) as above disclosed as the sole active ingredient or together with the at least one additional active ingredient can be used in combination with radiation therapy, surgery, immunotherapy besides other chemotherapies.
The term "pharmaceutically acceptable", including its variations, refers to compositions, carriers, diluents and reagents, eventually used interchangeably, useful for safe administration to or upon a mammal without the production of undesirable physiological effects such as nausea, dizziness, gastric upset and the like.

The pharmaceutical composition of the invention may be in liquid, semi-solid or solid form. The pharmaceutical composition of the invention in liquid form is preferably prepared in form of a solution, emulsion, lotion or dispersion.

The pharmaceutical composition of the invention in solid form is preferably prepared in form of capsules, tablets, pills, powders, dragees, microgranules or granules. Solid forms according to the invention are also suitable for solutions or suspensions in liquid form prior to use. The liquid preparation can also be emulsified, according to the invention prior to use.

The pharmaceutical compositions of the invention may be prepared according to known methods of manufacturing. Pharmaceutically acceptable excipient according to the invention may be selected among diluents, fillers, disintegrants, emulsifying agents, lubricants, stabilizers, surfactants, preservatives or a mixture thereof. For example, lactose, sodium citrate, calcium carbonate, dicalcium phosphate or a mixture thereof may be used as fillers and disintegrating agents such as starch, alginic acids and certain complex silicates combined with lubricants such as magnesium stearate, sodium lauryl sulphate, talc or a mixture thereof may be used for preparing tablets. To prepare a capsule, it is advantageous to use lactose and high molecular weight polyethylene glycols. In case of aqueous suspensions, emulsifying agents or agents which facilitate suspension may be usefully used to prepare the composition of the invention. Diluents such as sucrose, ethanol, polyethylene glycol, propylene glycol, glycerol, chloroform or a mixture thereof may also be used. The choice of vehicle or the excipient and the content of active ingredient in the vehicle or excipient are generally determined in accordance with the solubility and chemical properties of the active compound, the particular mode of administration and the provisions to be observed in pharmaceutical practice.

The pharmaceutical composition according to the invention can be administered in a suitable formulation to humans and animals by topical or systemic administration, including oral, rectal, nasal, buccal, ocular, sublingual, transdermal, rectal, topical, vaginal, parenteral (including subcutaneous, intra-arterial, intramuscular, intravenous, intradermal, intrathecal and epidural), intracisternal and intraperitoneal. The administration route may vary with the condition of the recipient.

The pharmaceutical formulation of the invention can be prepared in unit dosage form by any of the methods well known in the art of pharmacy. Such methods include the step of bringing into association the active ingredient with the carrier which constitutes one or more accessory ingredients. In general, the formulations are prepared by uniformly and intimately bringing into association the active ingredient with liquid carriers or finely divided solid carriers or both, and then, if necessary, shaping the product.

### A - EXPERIMENTAL PART and RESULTS

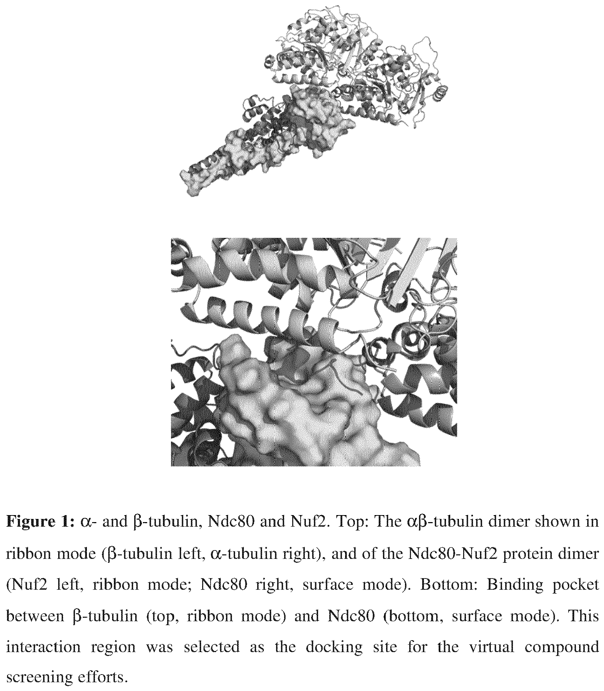

The inventors identified small molecule inhibitors of Ncd80 kinetochore subcomplex-MT binding by a virtual, computational-based, screening campaign. The virtual screening effort focused on the identification of chemical compounds that impair the ability of the Ndc80 kinetochore subcomplex to interact with β-tubulin. (Ciferri C. et al. (2008). Cell, 133:427-39) (Figure 1).

The 3-D structure of the Ndc80 kinetochore subcomplex bound to MTs was available at the PDB server signed with pdb code: 3IZ0 (Alushin, G.M. et al. (2010) Nature 467: 805-10). A commercially available compound library (www.specs.net) was docked at the interface between the Ndc80 protein and β-tubulin, and then sorted by pharmacophore model matching. The most promising compounds were visually inspected and ranked by binding free energy values (MM-GBSA). The best compounds were biochemically evaluated for their ability to physically prevent Ndc80 kinetochore subcomplex-MT binding *in vitro.*

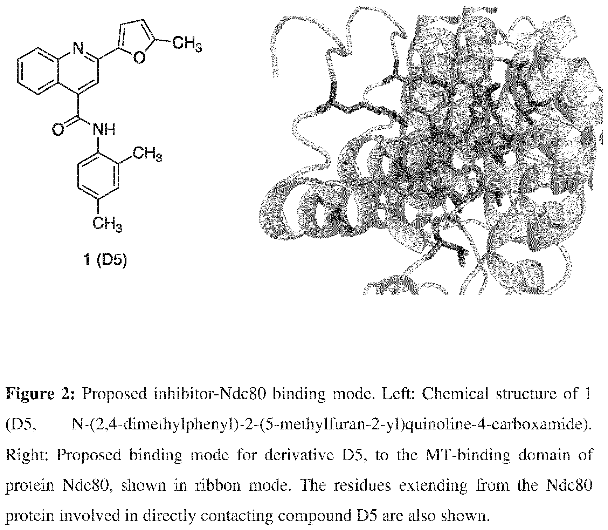

Among the tested compounds, derivative **1** (RS5982, D5) (Specs code: AK-968/15253415) showed the most promising result with an inhibition of 59.93 % in the biochemical AlphaScreen^{®} binding assay used. Inspection of the proposed binding mode, led to the identification of a series of crucial interactions: the dimethylphenyl group mediated a π-cation interaction with Arg872, and a hydrophobic contact with Ala951; the furane ring was at bond distance form Ile880; the quinoline function formed hydrophobic contacts with Leu 950, Thr965, His 968 and Ile969. (Figure 2).

Compounds of formula (I) were synthesised as depicted in Scheme 1, as below. 2-R₂-substituted quinoline-4-carboxylic acids were obtained by reaction of isatin with a R₂-ethanone derivative in the presence of 33% aqueous solution of potassium hydroxide in ethanol at 80 °C for 24 h, or, alternatively, under microwave (MW) irradiation at 300 W, from 25 °C to 80 °C in 2 min, and then for 5 min. at room temperature. The 2-R₂ substituted quinoline-4-carboxylic acids were transformed into the corresponding *N*-R₁ 2-R₂-substituted quinoline-4-carboxamides in *N,N-*dimethylformamide in the presence of triethylamine, (benzotriazol-1-yloxy)tripyrrolidinophosphonium hexafluorophosphate at 25 ° C for 16 h.

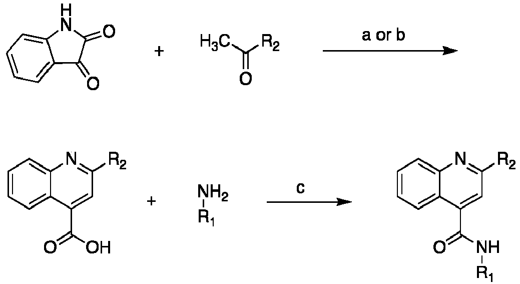

Scheme 1. Reagents and reaction conditions. (a) 33% KOH aqueous solution, ethanol, 80 °C, 24 h; (b) MW, 300 W, 25 °C to 80 °C in 2 min, room temp. 5 min (c) triethylamine, *N,N*-dimethylformamide, (benzotriazol-1-yloxy)tripyrrolidinophosphonium hexafluorophosphate, 25 ° C for 16 h.

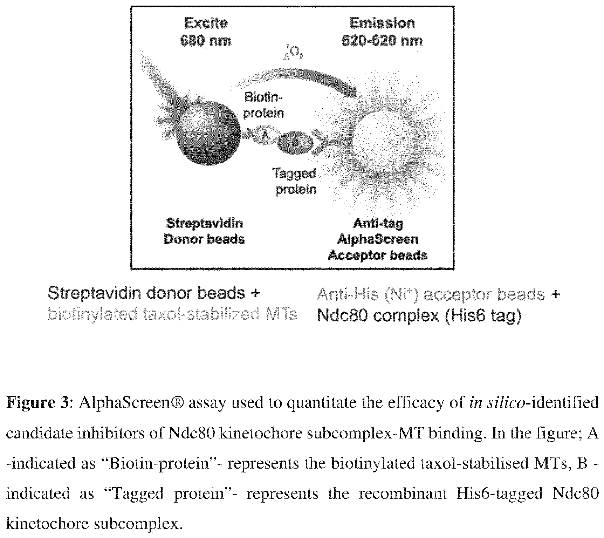

We carried out virtual screening studies to identify potential small molecule inhibitors of Ndc80 kinetochore subcomplex-MT binding at the interface of the Ndc80 kinetochore subcomplex and the α,β-tubulin dimer that it binds to (Ciferri C. et al. (2008). Cell, 133:427-39; Alushin G.M. et al. (2010). Nature, 467:805-10). *In silico*-identified inhibitory compounds were withheld based on their Gibbs free energy measurements, indicating inhibitor activity/potency, and tested biochemically for their ability to physically prevent Ndc80 kinetochore subcomplex-MT binding *in vitro* (Figure 3). Specifically, recombinant human Ndc80 kinetochore subcomplex (with the Ndc80 protein being His6-tagged) was produced in and purified from *E. coli* using anti-His-based affinity-purification and standard size-exclusion chromatography. Next, the Ndc80 kinetochore subcomplex was bound to AlphaScreen^{®} acceptor beads. In parallel, biotinylated and taxol-stabilised MTs were bound to streptavidin-covered AlphaScreen^{®} donor beads. Close binding between the Ndc80 kinetochore subcomplex and MTs resulted in the emission of 520-620 nm light by the acceptor beads following excitation of the donor beads with a 680 nm light source. A decrease in light emission is caused by abrogating Ndc80 kinetochore subcomplex-MT binding. Using this assay, the inventors confirmed the novel *in silico*-identified quinolone compounds as potent inhibitors of Ndc80 kinetochore subcomplex-MT binding (up to 60% efficacy as compared to control compound Cor55, which is an established inhibitor of Ndc80 kinetochore subcomplex-MT binding *in vitro* (Screpanti E., et al. (2010). PloS ONE, 5:e11603).

### B - MATERIALS AND METHODS

### Example 1. Synthesis of compounds of formula (I) according to the present invention.

Chemistry: All reagents and solvents were handled according to the material safety data sheet of the supplier and were used as purchased without further purification. Organic solutions were dried over anhydrous sodium sulphate. Evaporation of solvents was carried out on a Büchi Rotavapor R-210 equipped with a Büchi V-850 vacuum controller and a Büchi V-700 vacuum pump. Column chromatography was performed on columns packed with silica gel from Merck (70-230 mesh). Silica gel thin layer chromatography (TLC) cards from Merck (silica gel precoated aluminium cards with fluorescent indicator measurable at 254 nm) were used for TLC. Developed plates were visualised with a Spectroline ENF 260C/FE UV apparatus. Melting points (mp) were determined on a Stuart Scientific SMP1 apparatus and are uncorrected. Infrared (IR) spectra were recorded on a PerkinElmer Spectrum 100 FT-IR spectrophotometer equipped with a universal attenuated total reflectance accessory. IR data were acquired and processed by PerkinElmer Spectrum 10.03.00.0069 software. Band position and absorption ranges are given in cm⁻¹. Proton nuclear magnetic resonance (¹H NMR) spectra were recorded with a Varian Mercury (300 MHz) spectrometer in the indicated solvent, and the corresponding fid files were processed by MestreLab Research SL MestreReNova 6.2.1-769 software. Chemical shifts are expressed in δ units (ppm) from tetramethylsilane. Mass spectra were recorded on a Bruker Daltonics MicroTOF LC/MS mass spectrometer equipped with a positive ion ESI source.

The purity of tested compounds was checked by high pressure liquid chromatography (HPLC). Purity of tested compounds was found to be >95%. The Thermo Fisher Scientific Inc. Dionex UltiMate 3000 HPLC system consisted of an SR-3000 solvent rack, a LPG-3400SD quaternary analytical pump, a TCC-3000SD column compartment, a DAD-3000 diode array detector, and an analytical manual injection valve with a 20 µL loop. Samples were dissolved in acetonitrile (1 mg/mL). HPLC analysis was performed by using a Thermo Fisher Scientific Inc. Acclaim 120 C18 column (5 µm, 4.6 mm × 250 mm) at 25 ± 1 °C with an appropriate solvent gradient (acetonitrile/water), flow rate of 1.0 mL/min and signal detector at 206, 230, 254 and 365 nm. Chromatographic data were acquired and processed by Thermo Fisher Scientific Inc. Chromeleon 6.80 SR15 Build 4656 software.

The following examples are included to better disclose the invention without any limiting purpose.

### EXAMPLES

### Example 1.1: N-(2,4-Dimethylphenyl)-2-phenylquinoline-4-carboxamide (5) (RS5981).

To a solution of 2-phenylquinoline-4-carboxylic acid (0.250 g, 0.001 mol), 2,4-dimethylaniline (0.360 g, 0.003 mol), triethylamine (0.304 g, 0.004 mol) in *N,N-*dimethylformamide (5 mL) was added (benzotriazol-1-yloxy)tripyrrolidinophosphonium hexafluorophosphate (0.520 g, 0.001 mmol). The resulting mixture was stirred at 25 ° C for 16 h. then diluted with water and extracted with ethyl acetate. The organic layer was washed with brine, dried and filtered. Evaporation of the solvent gave a residue that was purified by column chromatography (silica gel, ethyl acetate:n-hexane= 9:1) to furnish RS5981 (0.150 g, 51%), mp 198-200 °C (from ethanol). ¹H NMR (DMSO-d₆, 300 MHz): δ 2.31 (s, 6H), 7.07-7.12 (m, 2H), 7.43-7.45 (m, 1H), 7.54-7.62 (m, 3H), 7.66-7.71 (m, 1H), 7.83-7.87 (m, 1H)8.15-8.25 (m, 2H), 8.34 (s, 1H), 8.35-8.37 (m, 2H), 10.24 ppm (br s, disappeared after treatment with D₂O, 1H). IR: v 1599 and 2982 cm⁻¹.

### Example 1.2: N-(2,4-Dimethylphenyl)-2-(5-methylfuran-2-yl)quinoline-4-carboxamide (1) (D5) (RS5982).

Was synthesised as RS5981 according to the process for example 1.1 starting from 2-(5-methylfuran-2-yl)quinoline-4-carboxylic acid and 2,4-dimethylaniline (RS5977). Yield 49%, mp 218-220 °C (from ethanol). ¹H NMR (DMSO-d₆, 300 MHz): δ 2.31 (s, 3H), 2.32 (s, 3H), 2.46 (s, 3H), 6.39-6.40 (m, 1H), 7.08-7.10 (m, 1H), 7.13-7.14 (m, 1H), 7.39 (d, *J* = 2.46 Hz, 1H), 7.43 (d, *J* = 6.0 Hz, 1H), 7.62-7.65 (m, 1H), 7.80-7.83 (m, 1H), 8.06 (s, 1H), 8.08 (d, *J* = 5.9 Hz, 1H), 8.15 (d, *J* = 5.9 Hz, 1H), 10.24 ppm (br s, disappeared after treatment with D₂O, 1H). IR: v 1621 and 2952 cm⁻¹.

### Example 1.3: N-(2,4-Dimethylphenyl)-2-(1H-pyrrol-2-yl)quinoline-4-carboxamide (4) (RS5989).

Was synthesised as RS5981 according to the process for example 1.1 starting from 2-(1H-pyrrol-2-yl)quinoline-4-carboxylic acid (RS5986) and 2,4-dimethylaniline. Yield 45%, mp 201-203 °C (from ethanol). ¹H NMR (DMSO-d₆, 300 MHz): δ 2.30 (s, 6H), 6.23-6.24 (m, 1H), 7.00-7.01 (m, 1H), 7.06-7.12 (m, 3H), 7.40-7.43 (m, 1H), 7.52-7.57 (m, 1H), 7.76 (t, *J* = 7.2 Hz, 1H), 7.99-8.02 (m, 1H), 8.06 (s, 1H), 8.06-8.10 (m, 1H), 10.19 (br s, disappeared after treatment with D₂O, 1H), 11.71 ppm (br s, disappeared after treatment with D₂O, 1H). IR: v 1580 and 3089 cm⁻¹.

### Example 1.4: N-(2-Hydroxyphenyl)-2-(5-methylfuran-2-yl)quinoline-4-carboxamide (10) (RS5994).

Was synthesised as RS5981 according to the process for example 1.1 starting from 2-(5-methylfuran-2-yl)quinoline-4-carboxylic (RS5977) acid and 2-aminophenol. Yield 50%, mp 242-245 °C (from ethanol). ¹H NMR (DMSO-d₆, 300 MHz): δ 2.44 (s, 3H), 6.38 (m, 1H), 6.84-6.89 (m, 1H), 6.92-6.95 (m, 1H), 7.05-7.08 (m, 1H), 7.34-7.35 (m, 1H), 7.57-7.62 (m, 1H), 7.76-7.81 (m, 2H), 8.04 (s, 1H), 8.04-8.06 (m, 1H), 8.15-8.17 (m, 1H), 9.97 ppm (br s, disappeared after treatment with D₂O, 2H). IR: v 1539 and 3121 cm⁻¹.

### Example 1.5: N-(2,4-Dimethylphenyl)-2-(thiophen-2-yl)quinoline-4-carboxamide (3) (RS5995).

Was synthesised as RS5981 according to the process for example 1.1 starting from 2-(thiophen-2-yl)quinoline-4-carboxylic acid (RS5984) and 2,4-dimethylaniline. Yield 61%, mp >300 °C (from ethanol). ¹H NMR (DMSO-d₆, 300 MHz): δ 2.30 (s, 6H), 7.06-7.11 (m, 1H), 7.24-7.27 (m, 1H), 7.44 (d, J = 8.1 Hz, 1H), 7.60-7.65 (m, 1H), 7.77-7.82 (m, 1H), 8.03 (d, J = 8.4 Hz, 1H), 8.13-8.15 (m, 1H), 8.30 (s, 1H), 10.22 ppm (br s, disappeared after treatment with D₂O, 1H). IR: v 1552 and 2987 cm⁻¹.

### Example 1.6: N-(2,4-Dimethylphenyl)quinoline-4-carboxamide (11) (RS6005).

Was synthesised as RS5981 according to the process for example 1.1 starting from quinoline-4-carboxylic acid and 2,4-dimethylaniline. Yield 85%, mp 133-134 °C (from ethanol). ¹H NMR (DMSO-d₆, 300 MHz): δ 2.27 (s, 3H), 2.28 (s, 3H), 7.04-7.10 (m, 2H), 7.36-7.38 (m, 1H), 7.67-7.70 (m, 1H), 7.73 (d, *J* = 3.9 Hz, 1H), 7.80-7.86 (m, 1H), 8.09-8.12 (m, 1H), 8.19-8.22 (m, 1H), 9.02 (d, *J* = 4.3 Hz), 10.17 ppm (br s, disappeared after treatment with D₂O, 1H). IR: v 1589 and 3005 cm⁻¹.

### Example 1.7: N-(2,4-Dimethylphenyl)-2-(furan-2-yl)quinoline-4-carboxamide (2) (RS6008).

Was synthesised as RS5981 according to the process for example 1.1 starting from 2-(furan-2-yl)quinoline-4-carboxylic acid (RS6003) and 2,4-dimethylaniline. Yield 59%, mp 204-205 °C (from ethanol). ¹H NMR (DMSO-d₆, 300 MHz): δ 2.29 (s. 6H), 6.76-6.77 (m, 1H), 7.06-7.11 (m, 2H), 7.41 (d, *J* = 8.1 Hz, 1H), 7.46-7.48 (m, 1H), 7.62-7.67 (m, 1H), 7.80-7.85 (m, 1H), 7.99 (s, 1H), 8.06-8.17 (m, 3H), 10.25 ppm (br s, disappeared after treatment with D₂O, 1H). IR: v 1523 and 2984 cm⁻¹.

### Example 1.8: N-(4-Hydroxyphenyl)-2-(5-methylfuran-2-yl)quinoline-4-carboxamide (9) (RS6017).

Was synthesised as RS5981 according to the process for example 1.1 starting from 2-(5-methylfuran-2-yl)quinoline-4-carboxylic acid (RS5977) and 2-aminophenol. Yield 15%, mp 268-270 °C (from ethanol). ¹H NMR (DMSO-d₆, 300 MHz): δ 2.42 (s, 3H), 6.35-6.36 (m, 1H), 6.76 (d, *J* = 8.7 Hz, 2H), 7.37 (d, *J* = 3.18, 1H), 7.55-7.58 (m, 3H), 7.75-7.80 (m, 1H), 8.00 (s, 1H), 8.02-8.05 (m, 2H), 9.34 (br s, disappeared after treatment with D₂O, 1H), 10.57 ppm (br s, disappeared after treatment with D₂O, 1H). IR: v 1511 and 3263 cm⁻¹.

### Example 1.9: N-(2,4-Dimethylphenyl)-2-(pyridin-4-yl)quinoline-4-carboxamide (7) (RS6020).

Was synthesised as RS5981 according to the process for example 1.1 starting from 2-(pyridin-4-yl)quinoline-4-carboxylic acid (RS6014) and 2,4-dimethylaniline. Yield 85%, mp 210-212 °C (from ethanol). ¹H NMR (DMSO-d₆, 300 MHz): δ 2.31 (s, 6H), 7.07-7.12 (m, 2H), 7.45 (d, *J* = 7.92 Hz, 1H), 7.72-7.77 (m, 1H), 7.87-7.92 (m, 1H), 8.20-8.28 (m, 2H), 8.30-8.32 (m, 2H), 8.46 (s, 1H), 8.80-8.82 (m, 2H), 10.24 ppm (br s, disappeared after treatment with D₂O, 1H). IR: v 1529 and 2981 cm⁻¹.

### Example 1.10: N-(2,4-Dimethylphenyl)-2-(pyridin-2-yl)quinoline-4-carboxamide (6) (RS6038).

Was synthesised as RS5981 according to the process for example 1.1 starting from (pyridin-2-yl)quinoline-4-carboxylic acid (RS6004) and 2,4-dimethylaniline. Yield 83%, mp 202-205 °C (from ethanol). ¹H NMR (DMSO-d₆, 400 MHz): δ 2.32 (s, 3H), 2.32 (s, 3H), 7.10 (d, *J* = 8.0 Hz), 7.14 (s, 1H), 7.41 (d, *J* = 5.9 H, 1H), 7.59-7.60 (m, 1H), 7.75-7.77 (m, 1H), 7.89-7.91 (m, 1H), 8.06-8.08 (m, 1H), 8.25 (dd, *J* = 7.8 and 12.9 Hz, 2H), 8.67 (d, *J* = 7.9 Hz, 1 H), 8.74 (s, 1H), 8.82-8.83 (m, 1H), 10.35 ppm (br s, disappeared after treatment with D₂O, 1H). IR: v 1504 and 2920 cm⁻¹.

### Example 1.11: 2-Phenylquinoline-4-carboxylic acid (RS5974).

A mixture of isatin (1.000 g, 0.007 mol), 33% KOH aqueous solution (3.4 mL), acetophenone (0.889 g, 0.008 mol) in ethanol (10.2 mL) was heated to 80 °C for 24 h. The solvent was concentrated in vacuum and the residue was dissolved by water, which was extracted with diethyl ether. The aqueous layer was acidified with glacial acid acetic to pH = 5. The resulting precipitate was filtered and dried to obtain RS5974 (1.55 g, 92%), mp 139-141 °C (from ethanol). ¹H NMR (DMSO-d₆, 300 MHz): δ 4.32 (br s, disappeared after treatment with D₂O, 1H), 7.53-7.62 (m, 4H), 7.75-7.80 (m, 1H), 8.08-8.10 (m, 1H), 8.24-8.27 (m, 3H), 8.60-8.62 ppm (m, 1H). IR: v 1652 and 2487 cm⁻¹.

### Example 1.12: 2-(1H-Pyrrol-2-yl)quinoline-4-carboxylic acid (RS5976).

Was synthesised as RS5974 (example 1.11) starting from isatin and 2-acetylpyrrole. Yield 52%, mp >300 °C (from ethanol). ¹H NMR (DMSO-d₆, 300 MHz): δ 3.71 (br s, disappeared after treatment with D₂O, 1H), 6.21-6.22 (m, 1H), 6.99-7.01 (m, 1H), 7.51-7.56 (m, 1H), 7.71-7.56 (m, 1H), 7.71-7.76 (m, 1H), 7.96-7.99 (m, 1H), 8.16 (s, 1H), 8.50-8.52 (m, 1H), 11.70 (br s, disappeared after treatment with D₂O, 1H). IR: v 1701 and 2497 cm⁻¹.

### Example 1.13: 2-(5-Methylfuran-2-yl)quinoline-4-carboxylic acid (RS5977).

Was synthesised as RS5974 (example 1.11) starting from isatin and 2-acetyl-5methylfuran. Yield 61%, mp 232-235 °C (from ethanol). ¹H NMR (DMSO-d₆, D₂O, 300 MHz): δ 2.42 (s, 3H), 6.35-6.36 (m, 1H), 7.32-7.33 (m, 1H), 7.59-7.65 (m, 1H), 7.76-7.81 (m, 1H), 8.04 (d, *J* = 8.4 Hz, 1H), 8.21 (s, 1H), 8.60 ppm (d, *J* = 8.4 Hz, 1H). IR: v 1698 and 2427 cm⁻¹.

### Example 1.14:2-(Thiophen-2-yl)quinoline-4-carboxylic acid (RS5984).

Was synthesised as RS5974 (example 1.11) starting from isatin and 2-acetylthiophene. Yield 65%, mp 2018-220 °C (from ethanol). ¹H NMR (DMSO-d₆, D₂O, 300 MHz): δ 7.22-7.25 (m, 1H), 7.62-7.67 (m, 1H), 7.77-7.83 (m, 2H), 8.02-8.10 (m, 2H), 8.41 (s, 1H), 8.55-8.58 ppm (m, 1H). IR: v 1720 and 2592 cm⁻¹.

### Example 1.15: 2-(Furan-2-yl)quinoline-4-carboxylic acid (RS6003).

Was synthesised as RS5974 (example 1.11) starting from isatin and 2-acetylfuran. Yield 100%, mp 214-215 °C (from ethanol). ¹H NMR (DMSO-d₆, 300 MHz): δ 6.75-6.76 (m, 1H), 7.44-7.45 (m, 1H), 7.64-7.69 (m, 1H), 7.80-7.85 (m, 1H), 7.97-7.98 (m, 1H), 8.06-8.09 (m, 1H), 8.28 (s, 1H), 8.62-8.65 (m, 1H), 13.01 ppm (br s, disappeared after treatment with D₂O, 1H). IR: v 1691 and 2235 cm⁻¹.

### Example 1.16: 2-(Pyridin-2-yl)quinoline-4-carboxylic acid (RS6004).

A mixture of isatin (1.000 g, 0.007 mol), 33% KOH aqueous solution (3.4 mL), acetophenone (0.889 g, 0.008 mol) in ethanol (10.2 mL) was placed was placed into a microwave cavity (closed vessel mode, P max 250 PSI). Microwave irradiation of 300 W was used, the temperature was raised from 25 °C to 80 °C in 2 min and the mixture was stirred at this temperature for 5 min. After cooling, the solvent was concentrated in vacuum and the residue was dissolved by water, which was extracted with diethyl ether. The aqueous layer was acidified with glacial acid acetic to pH = 5. The resulting precipitate was filtered and dried to obtain RS6004 (1.70 g, 99%), mp >300 °C (from ethanol). ¹H NMR (DMSO-d₆, 300 MHz): δ 7.53-7.57 (m, 1H), 7.71-7.76 (m, 1H), 7.84-7.90 (m, 1H), 8.01-8.06 (m, 1H), 8.18-8.21 (m, 1H), 8.62 (d, *J* = 8.0 Hz, 1H), 8.74-8.77 (m, 2H), 8.98 (s, 1H), 13.52 ppm (br s, disappeared after treatment with D₂O, 1H). IR: v 1701 and 2426 cm⁻¹.

### Example 1.17: 2-(Pyridin-4-yl)quinoline-4-carboxylic acid (RS6014).

Was synthesised as RS6004 (example 1.16) starting from isatin and 4-acetylpyridine. Yield 94%, mp 270-272 °C (from ethanol).¹H NMR (DMSO-d₆, 300 MHz): δ 7.72-7.77 (m, 1H), 7.86-7.91 (m, 1H), 8.19-8.25 (m, 3H), 8.53 (s, 1H), 8.64-8.67 (m, 1H), 8.76-8.77 (m, 2H), 13.80 ppm (br s, disappeared after treatment with D₂O, 1H). IR: v 1709 and 2342 cm⁻¹.

### 2. Computational studies

The bound Ndc80 kinetochore subcomplex:tubulin structure was downloaded from the PDB (www.rcsb.org), pdb code 3IZ0 (Alushin G.M. et al. (2010). Nature, 467:805-10). The tubulin dimer (chains A and B) together with the Ndc80-Nuf2 protein dimer were extracted from the protein model. Hydrogen atoms were added to the protein, using Maestro protein preparation wizard (G. M. Sastry, et al. (2013) J. Comput. Aid. Mol. Des. 27: 221-34) and minimised, keeping all the heavy atoms fixed until a rmsd gradient of 0.05 kcal/(mol·Å) was reached. The docking simulations were performed using PLANTS. (Korb, O. et al. (2009) J. Chem. Inf. Model. 49: 84-96.) It was settled a binding lattice of 12 Å radius using all default settings. The pharmacophore model was obtained by Phase, (S. L. Dixon, et al. (2006) Chem. Biol. Drug Des. 67: 370-2) fixing the queries at the hot spot residues in the Ndc80 protein. The commercially available compound library Specs (www.SPECS.net) was managed to filter out the compounds without drug-like properties, then docked (about 300,000 derivatives) at the Ndc80 protein-□-tubulin interface. All the docking proposed binding conformation were ranked by the pharmacophore matching and the 250 first ranked derivatives were visually inspected and submitted to MM-GBSA computation (Jacobson, M. P. et al. (2004) Proteins, 55, 351-67) by Maestro GUI. (Schrödinger Release 2015: Maestro, Schrödinger, LLC, New York, NY, www.schrodinger.com) Picture were done with PyMOL (PyMOL, DeLanoScientificLLC: SanCarlos, CA, USA.).

## Claims

1. A compound having the following formula (I): 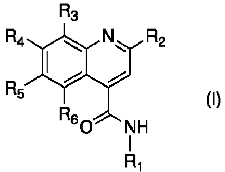 wherein
R₁ is selected among hydrogen, phenyl, heterocycle, benzofused heterocycle, optionally substituted at one or more position(s) with following same or different groups: alkyl, C₃-C₁₀ cycloalkyl, C₂-C₆ alkenyl, C₂-C₆ alkynyl, C₁-C₆ alkoxy, C₂-C₆ alkoxycarbonyl, 3-10 membered heterocycloalkyl, halogen, -CN, NH₂, OH, NO₂, NHR₁' NR₁'R₁" OR₁' where R₁', R₁" is alkyl, C₃-C₁₀ cycloalkyl, C₂-C₆ alkenyl, C₂-C₆ alkynyl, C₁-C₆ alkoxy, C₂-C₆ alkoxycarbonyl, 3-10 membered heterocycloalkyl or aromatic ring selected from phenyl, phenoxy, benzyl, benzyloxy, naphthyl, naphthyloxy, biphenyl or heterocycle, said aromatic ring optionally substituted at one or more position(s) with following same or different groups: alkyl, C₂-C₆ alkenyl, C₂-C₆ alkynyl, C₁-C₆ alkoxy, C₂-C₆ alkoxycarbonyl, 3-10 membered heterocycloalkyl, halogen, -CN, -NH₂, -OH, -NO₂;
R₂ is selected among hydrogen, phenyl, heterocycle, benzofused heterocycle, optionally substituted at one or more position(s) with following same or different groups: alkyl, C₃-C₁₀ cycloalkyl, C₂-C₆ alkenyl, C₂-C₆ alkynyl, C₁-C₆ alkoxy, C₂-C₆ alkoxycarbonyl, 3-10 membered heterocycloalkyl, halogen, -CN, NH₂, OH, NO₂, NHR₁' NR₁'R₁" OR₁' where R₁', R₁" is alkyl, C₃-C₁₀ cycloalkyl, C₂-C₆ alkenyl, C₂-C₆ alkynyl, C₁-C₆ alkoxy, C₂-C₆ alkoxycarbonyl 3-10 membered heterocycloalkyl or aromatic ring selected from phenyl, phenoxy, benzyl, benzyloxy, naphthyl, naphthyloxy, biphenyl or heterocycle, said aromatic ring optionally substituted at one or more position(s) with following same or different groups: alkyl, C₂-C₆ alkenyl, C₂-C₆ alkynyl, C₁-C₆ alkoxy, C₂-C₆ alkoxycarbonyl, 3-10 membered heterocycloalkyl, halogen, -CN, -NH₂, -OH, -NO₂;
R₃ is selected among hydrogen, alkyl, C₃-C₁₀ cycloalkyl, C₂-C₆ alkenyl, C₂-C₆ alkynyl, C₁-C₆ alkoxy, C₂-C₆ alkoxycarbonyl, 3-10 membered heterocycloalkyl, halogen, -CN, NH₂, OH, NO₂;
R4 is selected among hydrogen, alkyl, C₃-C₁₀ cycloalkyl, C₂-C₆ alkenyl, C₂-C₆ alkynyl, C₁-C₆ alkoxy, C₂-C₆ alkoxycarbonyl, 3-10 membered heterocycloalkyl, halogen, -CN, NH2, OH, NO₂;
R5 is selected among hydrogen, alkyl, C3-C10 cycloalkyl, C2-C6 alkenyl, C2-C6 alkynyl, C₁-C₆ alkoxy, C₂-C₆ alkoxycarbonyl, 3-10 membered heterocycloalkyl, halogen, -CN, NH₂, OH, NO₂.

2. A compound according to claim 1, in crystalline structure, polymorphic crystalline structure, pharmaceutically acceptable salt, racemate, diasteroisomer or enantiomer.

3. A compound according to claim 1 where the pharmaceutically acceptable salt is a hydrate salt.

4. A compound according to claim 1 where R1 is phenyl, heterocycle, benzofused heterocycle, optionally substituted at one or more position(s) with following same or different groups: alkyl, C₃-C₁₀ cycloalkyl, C₂-C₆ alkenyl, C₂-C₆ alkynyl, C₁-C₆ alkoxy, C₂-C₆ alkoxycarbonyl, 3-10 membered heterocycloalkyl, halogen, -CN, NH₂, OH, NO₂, NHR₁' NR₁'R₁" OR₁' thereof.

5. A compound according to claim 1 where R₂ is phenyl, heterocycle, benzofused heterocycle, optionally substituted at one or more position(s) with following same or different groups: alkyl, C₃-C₁₀ cycloalkyl, C₂-C₆ alkenyl, C₂-C₆ alkynyl, C₁-C₆ alkoxy, C₂-C₆ alkoxycarbonyl, 3-10 membered heterocycloalkyl, halogen, -CN, NH₂, OH, NO₂, NHR₁' NR₁'R₁" OR₁'.

6. A compound according to claim 1 where R₃ is selected among hydrogen, alkyl, C₃-C₁₀ cycloalkyl, C₂-C₆ alkenyl, C₂-C₆ alkynyl, C₁-C₆ alkoxy, C₂-C₆ alkoxycarbonyl, 3-10 membered heterocycloalkyl, halogen, -CN, NH2, OH, NO₂.

7. A compound according to claim I where R₄ is selected among hydrogen, alkyl, C₃-C₁₀ cycloalkyl, C₂-C₆ alkenyl, C₂-C₆ alkynyl, C₁-C₆ alkoxy, C₂-C₆ alkoxycarbonyl, 3-10 membered heterocycloalkyl, halogen, -CN, NH2, OH, NO₂.

8. A compound according to claim 1 where R₅ is selected among hydrogen, alkyl, C₃-C₁₀ cycloalkyl, C₂-C₆ alkenyl, C₂-C₆ alkynyl, C₁-C₆ alkoxy, C₂-C₆ alkoxycarbonyl, 3-10 membered heterocycloalkyl, halogen, -CN, NH2, OH, NO₂.

9. A compound according to claim 1 where R₆ is selected among hydrogen, alkyl, C₃-C₁₀ cycloalkyl, C₂-C₆ alkenyl, C₂-C₆ alkynyl, C₁-C₆ alkoxy, C₂-C₆ alkoxycarbonyl, 3-10 membered heterocycloalkyl, halogen, -CN, NH2, OH, NO₂.

10. A compound of formula (I) according to anyone of the preceding claims, which has one of the following formulae:

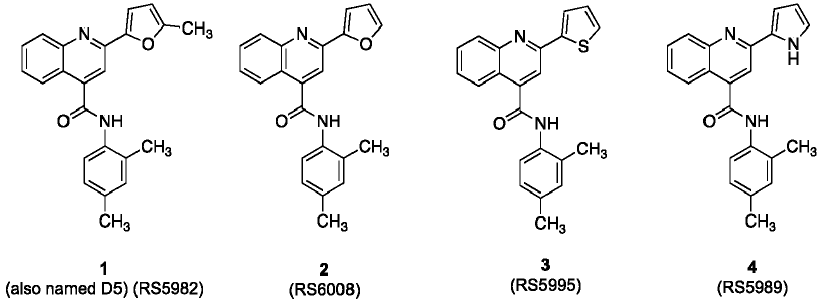

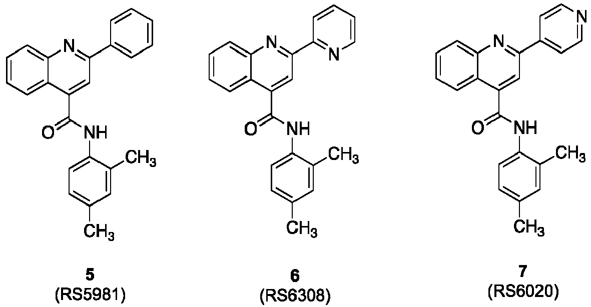

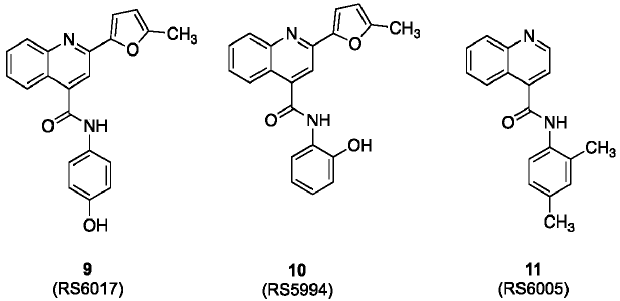

11. A compound of formula (I) according to anyone of the preceding claims, which has the following formula:

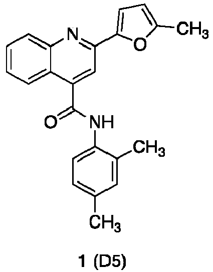

12. Pharmaceutical composition comprising at least one compound according to claims 1 to 11 in combination with at least one pharmaceutically acceptable excipient.

13. A compound according to anyone of the claims 1-11 or a pharmaceutical composition according to claim 12 for use as a medicament

14. A compound according to anyone of the claims 1-11 or a pharmaceutical composition according to claim 12 for use as inhibitor of Ndc80 kinetochore subcomplex-MT and kinetochore-MT binding.

15. A compound according to any one of the claims 1-11 or a pharmaceutical composition according to claim 12 for use in the treatment and/or prevention of cell proliferative disorders.

16. A compound according to any one of the claims 1-11 or a pharmaceutical composition according to claim 12 for use in the treatment of cancers and/or multi drug resistant cancers.

17. A compound according to any one of the claims 1-11 or a pharmaceutical composition according to claim 12 for use in the treatment of breast cancer, uterine cancer, brain cancer, lung cancer, liver cancer, kidney cancer, testicular cancer, pancreatic cancer, skin cancer, thyroid cancer, intestinal cancer, head and neck cancer, ovarian cancer, stomach cancer, colon cancer, bladder cancer, prostate cancer and urinary tract cancer, eye cancer, cancer of the central nervous system, oral and oropharyngeal cancers, HIV/AIDS-related cancers, hematopoietic and lymphoid cancers, and sarcomas of various tissues.
